# EUROPEAN PATENT SPECIFICATION

(11) **EP 3 936 099 B1**
(45) Date of publication and mention of the grant of the patent: **30.10.2024**
(21) Application number: 20184425.5
(22) Date of filing: 07.07.2020
(51) Int. Cl.: A61F 13/15, A61F 13/51, A61F 13/514

(54) **MANUFACTURING METHOD FOR AN ABSORBENT ARTICLE COMPRISING A POCKET AND ABSORBENT ARTICLE BEING PRODUCIBLE THEREBY**
HERSTELLUNGSVERFAHREN FÜR EINEN SAUGFÄHIGEN ARTIKEL MIT EINER TASCHE UND DADURCH HERSTELLBARER SAUGFÄHIGER ARTIKEL
PROCÉDÉ DE FABRICATION D'UN ARTICLE ABSORBANT COMPRENANT UNE POCHE ET ARTICLE ABSORBANT ÉTANT AINSI PRODUIT

(43) Date of publication of application: 12.01.2022
(73) Proprietor: Ontex BV, 9255 Buggenhout (BE); Ontex Group NV, 9320 Erembodegem (BE)
(72) Inventor: HELLMOLD, Jens, 59269 Beckum (DE); HEIRMAN, Lisa, 9250 Waasmunster (BE)
(74) Representative: Saurat, Thibault

(56) References cited:
- EP-A1- 3 213 727
- WO-A1-2012/126507
- WO-A1-98/04225
- WO-A2-2004/049992

## Description

The invention relates to a manufacturing method for manufacturing an absorbent article comprising a pocket being accessible from the garment facing side of the absorbent article and an absorbent article being producible by the steps of the manufacturing method.

Generally, in times of an increasing number of elderly and care-dependent people, there is a growing need of a manufacturing method for an absorbent article comprising a pocket and an absorbent article being producible thereby. In this context, for instance, a wetness monitoring device or any kind of a sensor may be attached to the absorbent article with the aid of the pocket in order to handle incontinence in a particularly efficient manner.

WO 2018/229121 A1 relates to a substrate suitable for incorporation into an absorbent article for automatic detection of wetness events therein. The substrate comprises a first surface capable of being arranged proximal to a body facing side of the absorbent article and a second surface opposite said first surface and capable of being arranged proximal to a garment facing side of said absorbent article. The substrate comprises a plurality of sensor tracks disposed on said first surface, wherein said sensor tracks are in electrical communication with a clip-on data processing module when connected at a position proximal to a first end of the substrate such to form a closed electrical circuit. The substrate comprises one or more slits and an insulating layer placed over said first surface to sandwich said sensor tracks therebetween. A pocket is formed between said first surface and said insulating layer proximal to at least said first end. The pocket is in fluid communication with said slit(s) and arranged to retain at least a portion of said clip-on data processing module therein. Disadvantageously, with special respect to manufacturing, said document does not show how the different elements such as the slits and the layers can properly be aligned in a highly efficient and accurate manner. EP3213727 also discloses an absorbent article having a pocket for holding an electrical device.

Accordingly, there is an object to provide a manufacturing method for manufacturing an absorbent article comprising a pocket being accessible from the garment facing side of the absorbent article and an absorbent article being producible by the steps of the manufacturing method, whereby both a particularly high efficiency and a notably high accuracy are ensured.

This object is solved by the features of the first independent claim for a manufacturing method for manufacturing an absorbent article comprising a pocket being accessible from the garment facing side of the absorbent article and the features of the second independent claim for an absorbent article being producible or produced by the steps of the manufacturing method. The dependent claims contain further developments. According to a first aspect of the invention, a manufacturing method for manufacturing an absorbent article comprising a backsheet layer on the garment facing side of the absorbent article and a pocket being accessible from the garment facing side of the absorbent article through an opening of the backsheet layer is provided. Said manufacturing method comprises the steps of providing a backsheet layer, a cover strip layer, and an absorbent core layer, cutting an opening into the backsheet layer, and placing the cover strip layer between the backsheet layer and the absorbent core layer, in such a manner that the opening of the backsheet is at least partly covered by the cover strip layer. In this context, the cutting is speed-synchronized with respect to the provision of the backsheet layer in such a manner that the opening is placed at the same position for every absorbent article especially in a sequence of absorbent articles.

Alternatively, in the case that the absorbent article further comprises a pattern on the backsheet layer, preferably on the body, especially the human body, facing side of the backsheet layer, the backsheet layer is speed-controlled with respect to the positioning of the pattern in such a manner that the opening is placed at the same position relatively to the pattern for every absorbent article especially in a sequence of absorbent articles. Advantageously, a particularly high efficiency and accuracy can be ensured.

According to a first preferred implementation form of the first aspect of the invention, the pattern is arranged in such manner onto the backsheet layer that the same portion of the pattern is positioned in the same way for every absorbent article.

Additionally or alternatively, the pattern comprises a conductive pattern especially being applied onto the backsheet layer by means of printing and/or spraying and/or laminating conductive material onto the backsheet layer and/or a non-conductive pattern especially being applied onto the backsheet layer (11) by means of printing and/or embossing.

It is further noted that at least one, preferably each, of the backsheet layer, the cover strip layer, and the absorbent core layer may especially be provided with the aid of an unwinder. Advantageously, for instance, the different layers can be provided in a highly efficient manner.

According to a second preferred implementation form of the first aspect of the invention, the pattern comprises an ink, preferably a carbon-based ink and/or a conductive polymer-based ink. In this context, the ink especially comprises at least one of graphene, graphite, nano-carbon-tubes, polyacetylene, polypyrrole, polyaniline and copolymers thereof, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), poly(3,4-ethylenedioxythiophene) polystyrene sulfonate (PEDOT:PSS), or any combination thereof.

According to a further preferred implementation form of the first aspect of the invention, the pattern is applied onto the backsheet layer prior to converting the backsheet layer. As an alternative, the pattern is applied within the same or another manufacturing process onto the backsheet layer with the aid of a pattern application unit. In this context, the pattern application unit is especially synchronized at least with the process step of cutting the opening into the backsheet layer.

It is noted that the pattern application unit may especially comprise at least one of rotary flexo printing, rotary gravure printing, ink jet printing, a hotmelt application with conductive glue especially in the context of contact coating or spraying, or any combination thereof.

According to a further preferred implementation form of the first aspect of the invention, with the aid of a visual control unit, the positioning of the pattern on the backsheet layer relatively to the opening of the backsheet layer is visually controlled to determine a first offset value and/or a second offset value by processing visual inspection data. In this context, the first offset value is especially adapted to quantify the deviation of said positioning in converting direction of the backsheet layer. In addition to this or as an alternative, the second offset value is especially adapted to quantify the deviation of said positioning in the direction perpendicular to the converting direction of the backsheet layer. Further additionally or further alternatively, the visual control unit especially comprises a camera and/or an optical sensor.

According to a further preferred implementation form of the first aspect of the invention, the first offset value is used to drive the speed-control of the backsheet layer.

According to a further preferred implementation form of the first aspect of the invention, the second offset value is used to move the backsheet layer with the pattern relatively to the opening and relatively to the cover strip layer in a direction perpendicular to the converting direction in such a manner that the second offset value is reduced towards zero. In this context, the relative positioning of the backsheet layer is especially driven by means of a web guiding device for the backsheet layer and/or by a web guiding device for the cover strip layer and/or by a device to move a corresponding cutting device for cutting the opening in a direction perpendicular to the converting direction.

According to a further preferred implementation form of the first aspect of the invention, the opening of the backsheet layer is cut with the aid of at least one of a rotary knife, a cutting die, a punching tool, a laser cutting tool, a water jet cutting tool, or any combination thereof. In addition to this or as an alternative, the cover strip layer is placed between the backsheet layer and the absorbent core layer in such a manner that the opening of the backsheet layer is at least partly covered by the cover strip layer. Advantageously, for example, the opening can efficiently and accurately be produced.

According to a further preferred implementation form of the first aspect of the invention, the width of the cover strip layer is less than the width of the backsheet layer and/or the width of the absorbent article. In addition to this or as an alternative, the width of the cover strip layer is wider than the opening of the backsheet layer. Further additionally or further alternatively, the cover strip layer comprises or is a liquid barrier. Advantageously, for instance, leakproofness can efficiently be ensured.

According to a further preferred implementation form of the first aspect of the invention, the cover strip layer is non-intermittently placed. As an alternative, the cover strip layer is intermittently placed. In this context, the intermittent placement of the cover strip layer comprises the additional step of cutting the cover strip layer in such a manner that the length of the cover strip layer is less than the length of the backsheet layer and/or the length of the absorbent article. The intermittent placement of the cover-strip is preferably done by means of a cut-and-place unit. Advantageously, for example, material can be saved, which leads to an increased efficiency.

According to a further preferred implementation form of the first aspect of the invention, the manufacturing method comprises the step of sealing at least two edges, preferably four edges, of the cover strip layer to the backsheet layer especially in such a manner that the sealing pattern is surrounding the opening to form the pocket between said backsheet layer and said cover strip layer. Advantageously, for instance, the size of the pocket can efficiently and accurately be limited according to a desired purpose.

According to a further preferred implementation form of the first aspect of the invention, the step of sealing comprises an adhesive application and/or a welding, preferably an ultrasonic welding, application. Advantageously, for example, sealing can be achieved in a cost-saving manner.

According to a further preferred implementation form of the first aspect of the invention, the backsheet layer comprises at least one outer layer and at least one inner layer being closer to the cover strip layer than the at least one outer layer. In this context, at least two, preferably all, layers of the backsheet layer are especially laminated to each other. In addition to this or as an alternative, the at least one outer layer especially covers at least a part of the surface of the at least one inner layer and/or especially at least surrounds the opening of the backsheet layer.

Advantageously, especially to reduce the coverage in converting direction, the layer may be intermittently applied preferably by means of a cut-and-place unit. Further advantageously, especially to reduce the coverage in a direction perpendicular to the converting direction the outer layer maybe be chosen with a width less than the width of the inner layer preferably with the infeed of said outer layer with reduced width from an unwinder.

According to a further preferred implementation form of the first aspect of the invention, the at least one outer layer comprises a substrate, preferably a non-woven substrate. In addition to this or as an alternative, the at least one inner layer comprises a film, preferably a non-permeable film. Advantageously, for example, the backsheet or the properties thereof, respectively, provide different benefits with special respect to the usage scenario that the absorbent article is used as a diaper.

In the following, some embodiments of the first aspect are noted, which can be combined with the preferred implementation forms described above in an additional manner or an alternative manner, respectively.

With respect to the above-mentioned at least one inner layer, it might be particularly advantageous if the at least one inner layer, preferably the film, more preferably the non-permeable film, is configured to be breathable. Advantageously, for instance, skin irritations can be reduced.

Furthermore, it might be particularly advantageous if the manufacturing method comprises the step of laminating the at least one outer layer and the at least one inner layer in order to form the backsheet layer. Advantageously, for example, the backsheet can be produced in a highly efficient manner. Further advantageously, if the outer layer comprises a non-woven substrate, it will especially form a textile backsheet, preferably giving the garment facing side a fabric like appearance.

Moreover, as partly noted above, the backsheet layer may comprise the pattern, preferably a printed and/or embossed pattern. As an alternative, in the case of the at least one inner layer, the at least one inner layer may comprise the pattern, preferably a printed and/or embossed pattern. Further alternatively, in the case of the film, the film, preferably the non-permeable film, may comprise the pattern or a printed and/or embossed pattern, respectively. In this context, the pattern or the printed and/or embossed pattern is especially printed and/or embossed in such a manner that the same portion of the pattern or the printed and/or embossed pattern is positioned in the same way for every absorbent article. Advantageously, for instance, with the aid of the printed and/or embossed pattern, elements of the absorbent article can efficiently and accurately be positioned in the context of a production line.

It is further noted that it might be particularly advantageous if the manufacturing method comprises the steps of monitoring the pattern, preferably the printed and/or embossed pattern, and controlling the manufacturing, preferably the manufacturing speed, more preferably parts of a respective production line being in charge of the manufacturing speed, most preferably parts of a respective production line being in charge of the manufacturing speed separately with regard to each other, on the basis of the respective monitoring result in such a manner that at least one, preferably each, of the opening, the cover strip layer, and the absorbent core layer remains relatively to the pattern, preferably the printed and/or embossed pattern, in the same position. Advantageously, for example, an undesired shift against a predefined target position especially in machine direction of a production line can efficiently and accurately be corrected. It is noted that said machine direction can also be called or understood as converting direction.

Furthermore, the manufacturing method may comprise the steps of identifying the cross-directional position of the pattern, preferably the printed and/or embossed pattern, relatively to the corresponding general center line of the backsheet layer, and controlling the cross-directional position of the backsheet layer on the basis of the respective identifying result. Advantageously, for instance, an undesired shift in the direction perpendicular to the machine direction of a production line within the plane of the absorbent article can be corrected in a highly efficient and accurate manner.

It might be particularly advantageous if the manufacturing method comprises the steps of recognizing the edges of the backsheet layer and/or the pattern, and controlling the cross-directional position of the backsheet layer on the basis of the respective recognizing result. Advantageously, for example, complexity can be reduced by using a web guide, which leads to an increased efficiency.

According to a second aspect of the invention, an absorbent article comprising a backsheet layer, a cover strip layer, an absorbent core layer, and a pocket being accessible from the non-absorbent side of the absorbent article is provided. Said absorbent article is producible by the steps of a method of one of the implementation forms of the first aspect of the invention. In said article the width of the cover strip layer is less than the width of the backsheet layer and/or the width of the absorbent article, and/or wherein the width of the cover strip layer is wider than the opening of the backsheet layer, and wherein the cover strip layer comprises or is a liquid barrier. Alternatively, said absorbent article is produced by the steps of a method of one of the implementation forms of the first aspect of the invention. Advantageously, a particularly high efficiency and accuracy can be ensured.

Exemplary embodiments of the invention are now further explained with respect to the drawings by way of example only, and not for limitation. In the drawings:
- Fig. 1: shows a flow chart of an exemplary embodiment of the inventive manufacturing method;
- Fig. 2: shows a first exemplary embodiment of a production line in accordance with the invention;
- Fig. 3: shows a second exemplary embodiment of a production line in accordance with the invention;
- Fig. 4: shows four exemplary embodiments of forming the pocket by sealing the cover strip to the backsheet in accordance with the invention;
- Fig. 5: shows four exemplary cases of alignment or misalignment with respect to the elements of the absorbent article;
- Fig. 6: shows an exemplary embodiment of an absorbent article with a pattern of conductive and non-conductive elements in combination in accordance with the invention; and
- Fig. 7: shows a flow chart of an exemplary embodiment of the inventive manufacturing method in greater detail.

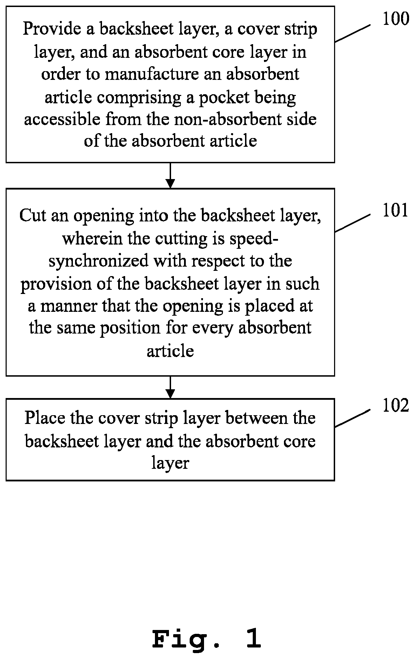

Firstly, Fig. 1 shows a flow chart of an embodiment of the inventive manufacturing method. According to a first step 100, the method comprises providing a backsheet layer, a cover strip layer, and an absorbent core layer in order to manufacture an absorbent article comprising a pocket being accessible from the non-absorbent side of the absorbent article. Then, according to a second step 101, the method comprises cutting an opening into the backsheet layer. In this context, the cutting is speed-synchronized with respect to the provision of the backsheet layer in such a manner that the opening is placed at the same position for every absorbent article. Furthermore, according to a third step 102, the method comprises placing the cover strip layer between the backsheet layer and the absorbent core layer.

With respect to the absorbent article, especially the pocket thereof, it is noted that such an absorbent article may exemplarily be used within the following usage scenario:
Equipping an absorbent article with an integrated sensor and providing an electronic device that needs to be attached to the product to allow the read-out of sensor data. The electronic device needs to be in conductive interconnection to the printed sensor inside the absorbent article and it needs to be securely attached to allow a reliable interconnection and to be protected against unintended loss or removal of the device. For this purpose, the absorbent article may preferably comprise the pocket, wherein at least a part of the electronic device can be inserted and that allows a reliable attachment of the device.

Generally, the invention is about the process that allows the formation of the pocket for an absorbent article on a scale of mass production. For instance and as an advantage, the construction may especially allow the conductive interconnection between the inserted device and the printed sensor of the absorbent article. Further advantageously, the construction is cost efficient and the manufacturing method or process, respectively, does not sacrifice the speed of a production line.

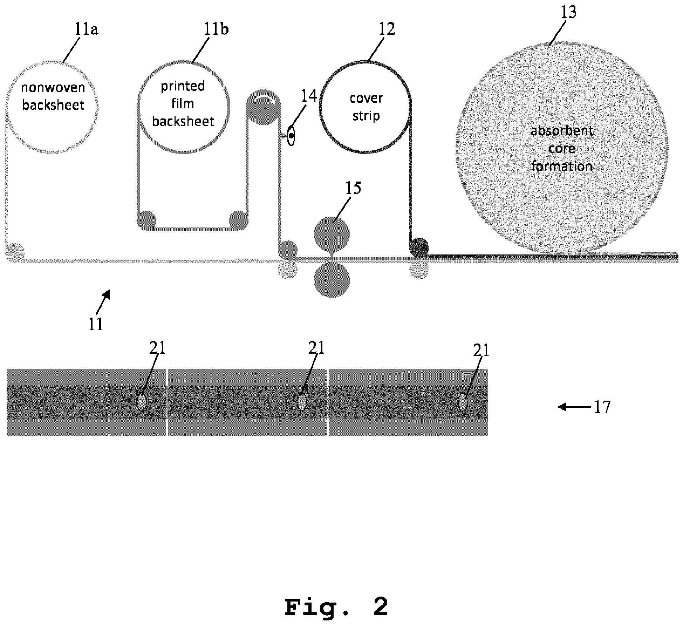

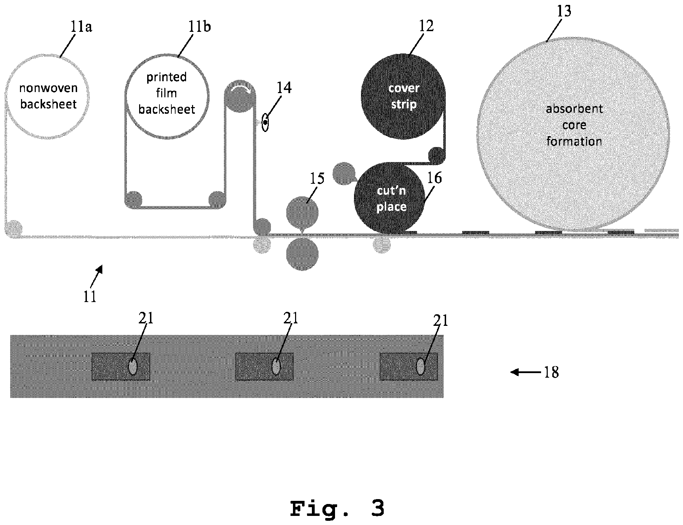

Now, the manufacturing method will be explained in greater detail with the aid of the first embodiment of a production line according to Fig. 2 and the second embodiment of a production line according to Fig. 3 in the following. In this context, it might particularly advantageous if at least one, exemplarily each, of the backsheet layer 11, the cover strip layer 12, and the absorbent core layer 13 is provided with the aid of an unwinder as illustrated by Fig. 2 and Fig. 3.

As it can further be seen from Fig. 2 or Fig. 3, respectively, the process is adding the layer with the cover strip 12 that comes from the corresponding unwinder and that is sealed to the inner side of the backsheet 11, so that the cover strip is placed between the backsheet 11 and the absorbent core 13 (or any other layer that is underneath the backsheet and above the core). In this context, the width of the cover strip 12 is less than the full width of the product or the absorbent article, respectively, but is wider than the opening of the pocket so that, for example, the above-mentioned device fits into the pocket and is surrounded by it, at least partially.

With respect to the first and the second embodiment of a production line, it is noted that the cover strip or the cover strip layer 12, respectively, can be added with the full length of the product or the absorbent article, respectively (non-intermittent application according to Fig. 2). As an alternative, the cover strip or the cover strip layer 12, respectively, can have a shorter length when it is applied by a cut-and-place application 16 (intermittent application according to Fig. 3).

It is further noted that said non-intermittent application is especially illustrated by the top view 17 of the product or the absorbent article, respectively, which is shown in Fig. 2, whereas said intermittent application is especially elucidated with the aid of the top view 18 of the product or the absorbent article, respectively, which is shown in Fig. 3. In addition to this, said top views 17, 18 especially illustrate the position for the opening 21.

Generally, the opening of the backsheet layer 11 is cut with the aid of at least one of a rotary knife, a punching tool, a laser cutting tool, a water jet cutting tool, or any combination thereof. In the case of both Fig. 2 and Fig. 3, the opening is exemplarily cut with the aid of a cutting die 15. Furthermore, as approximately mentioned above, the cover strip layer 12 may be placed between the backsheet layer 11 and the absorbent core layer 13 in such a manner that the opening of the backsheet layer 11 is at least partly covered by the cover strip layer 12.

Moreover, as roughly mentioned above, it might be particularly advantageous if the width of the cover strip layer 12 is less than the width of the backsheet layer 11 and/or the width of the absorbent article 13. In addition to this or as an alternative, the width of the cover strip layer 12 may be wider than the opening of the backsheet layer 11. Further additionally or further alternatively, the cover strip layer 12 comprises or is a liquid barrier.

In the case of both the first embodiment of a production line according to Fig. 2 and the second embodiment according to Fig. 3, the cover strip 12 is sealed to the backsheet 11, after creating the opening in the backsheet 11, by applying a glue pattern on the cover strip 12 or on the backsheet 11 (but preferably on the cover strip 12) through an adhesive application or by means of an ultrasonic welding unit or by a combination thereof.

The sealing pattern preferably creates a liquid barrier by sealing the edges of the cover strip 12 and especially by sealing the front- and/or backend of the strip 12 so that in between a pocket area is formed. Such a sealing at front and/or back can create benefits, for example, to have a precise control on how deeply an element such as the above-mentioned device can be inserted (downwards), or to ensure that an element such as said device cannot be inserted in the wrong direction (upwards).

Alternatively, in case of an non-intermittent cover strip 11 or in case of a cover strip 11 that extends well enough beyond the edges of the absorbent core 13 of the product, the sealing at front and back may be left out because they are not needed to create a liquid barrier in that case. It is noted that for a combination of different intermittent glue traces, a triple shim or multi shim glue applicator can be used. Additionally or alternatively, the subsequent arrangement of standard glue applicators can be used for the same purpose.

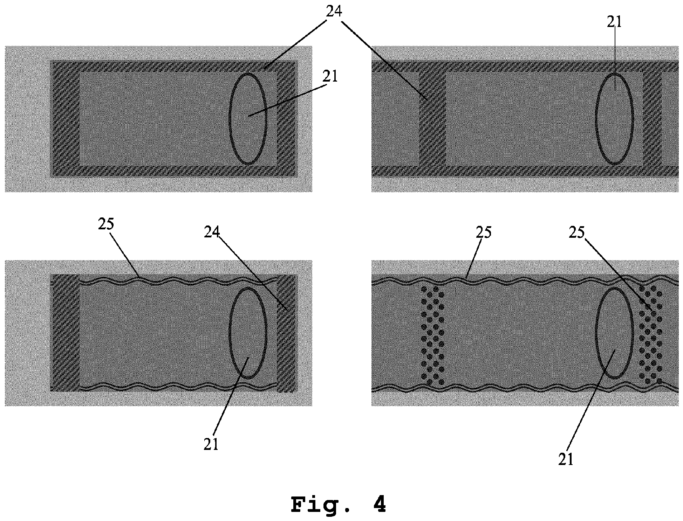

With respect to the above-mentioned sealing, Fig. 4 shows four exemplary embodiments of forming the pocket by sealing the cover strip 12 to the backsheet 11. In this context, each of said four embodiments illustrates the position for the opening 21. The top left embodiment comprises a glue pattern 24 at every edge (more specifically, front, back, and two side edges) especially for the intermittent case, whereas the top right embodiment comprises a glue pattern 24 at every edge (more specifically, front, back, and two side edges) especially for the non-intermittent case.

Furthermore, the bottom left embodiment of Fig. 4 comprises a glue pattern 24 at two edges (more specifically, front and back) and a welding pattern 25, preferably an ultrasonic welding pattern, at the other two edges (more specifically, two side edges) especially for the intermittent case, whereas the bottom right embodiment comprises a welding pattern 25, preferably an ultrasonic welding pattern, at every edge (more specifically, front, back, and two side edges).

With respect to said four embodiments according to Fig. 4, it is noted that glue and welding patterns can interchangeably be applicated with regard to each other.

Again, with respect to Fig. 2 and Fig. 3, the backsheet 11 comprises at least one outer layer with a non-woven substrate 11a and at least one inner layer with a non-permeable film 11b. The film can have breathable properties and it might be printed (both optionally). The laminate of at least one inner layer and at least one outer layer can be created with an inline lamination process.

Alternatively, the lamination process can be managed in advance, so that the pre-laminated backsheet can be used only on the unwinder for the backsheet film. Further alternatively, it is noted that the backsheet may comprise only one layer which is at least a non-permeable film, preferably a liquid-impermeable film.

As it can further be seen from both Fig. 2 and Fig. 3, the opening of the pocket is created by the above-mentioned cutting die 15 that follows the backsheet lamination process step (if applicable) and that is placed before the lamination step of the cover strip 12. Through the opening the pocket especially becomes accessible from the outer side of the absorbent article 13. The opening is especially placed at the same position for every product or absorbent article, respectively, by speed synchronization of the cutting die 15.

As an alternative to the creation of a pocket with an opening at any position within the absorbent article, preferably a diaper, a pocket can be created in the same manner through sealing left, right (and bottom) edges of the cover strip, but with its front edge not sealed and aligned with the front edge of the diaper. This avoids the need for an additional knife installation, but it may have the disadvantages that there is no freedom for where to position the pocket (it can only be accessed at the edge of the product) and that it may be difficult to separate the cover strip from the backsheet to access the pocket (as these thin layers typically stick together by static electricity).

As a further alternative to the creation of a pocket with an opening as imagined above, the entire part of a backsheet above the intended location of the opening may be removed, leaving only the cover strip as a barrier between the absorbent core and the outside of the product or the absorbent article, respectively, which may lead to several disadvantages.

Again, with respect to the first embodiment of a production line according to Fig. 2 and the second embodiment according to Fig. 3, it is noted that the backsheet may be printed and/or embossed and the design or pattern, respectively, may preferably be registered with a certain repeat length, especially aiming to have the same portion of the print design positioned in the same way for every single product or absorbent article, respectively, in machine direction. In this context, said machine direction is especially defined as the direction of the production process.

This may preferably require a registration control. In accordance with Fig. 2 and Fig. 3, the print or the printed and/or embossed pattern, respectively, of the backsheet 11 is monitored by a monitoring device, exemplarily an optical sensing unit 14 such as an eyemark sensor or a camera system. The output of that monitoring device is preferably used to control the speed of a pull roller, so that the speed of that driven pull roller can increase or decrease to shift the print design of the printed film to the right position. The right position ensures that also the other recurrent design elements remain relatively to the print design in the same position; this especially applies to the opening, to the sealing pattern of the pocket and to the position of the absorbent core.

Furthermore, the printed backsheet may also be controlled to stay in the right position in cross-direction. In this context, said cross-direction is especially defined as the direction perpendicular to the machine direction, within the plane of the product or the absorbent article, respectively. A device being configured to control the position of the respective web in cross-direction such as a web guide can control and adjust the position of the substrate according to the edges of the substrate.

Alternatively, the web guide or said device, respectively, can control and adjust the position of the substrate according to the print on that substrate by using an identification device such as a camera system that identifies the cross-directional position of the print relatively to the general center line. This method is advantageous because a print may sometimes not be exactly centered against the edges of its substrate. The web-guiding element ensures that the print in cross-direction remains in the same position relatively to the arrangement of other elements, such as the sealing pattern of the pocket or the position of the opening to said pocket.

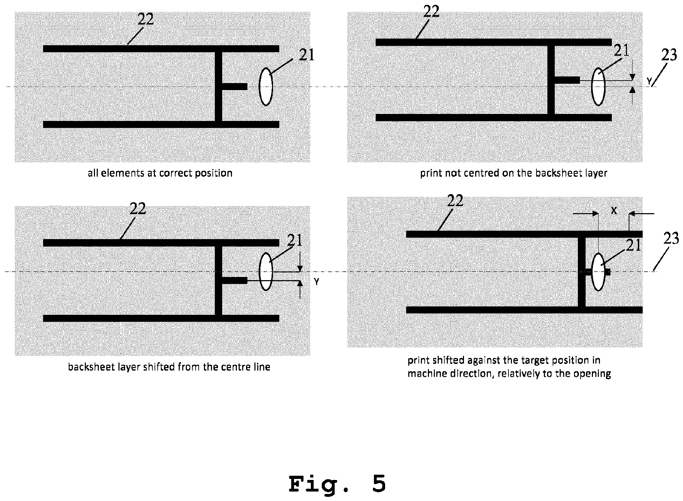

Moreover, with respect to the above-mentioned printed and/or embossed pattern, Fig. 5 shows said pattern 22 or design, respectively, within the scope of four exemplary cases of alignment or misalignment with respect to the elements of the product or the absorbent article, respectively. In this context, each of said four embodiments illustrates the position for the opening 21 and the general center line 23.

As it can be seen from Fig. 5, according to the top left embodiment, all elements of the product are at correct position, whereas in accordance with the top right embodiment, the printed and/or embossed pattern 22 is not centered on the substrate. Furthermore, according to the bottom left embodiment, the substrate is shifted from the center line 23, whereas the printed and/or embossed pattern 22 is shifted against the target position in machine direction in accordance with the bottom right embodiment of Fig. 5.

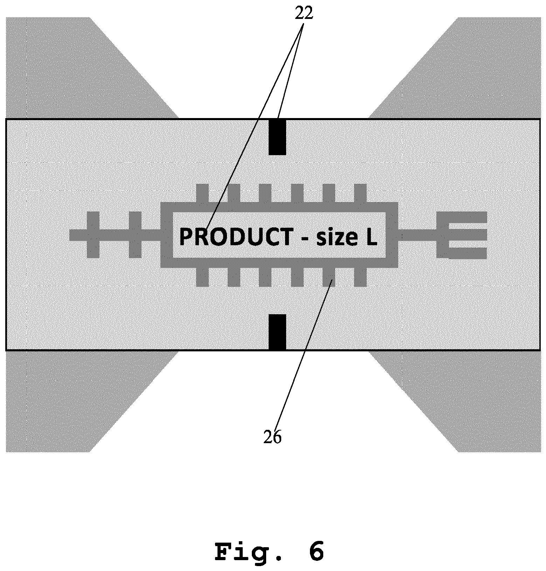

Now, with respect to Fig. 6, an exemplary embodiment of an absorbent article, especially in the form of a diaper, in accordance with the invention is illustrated.

Exemplarily, according to Fig. 6, the inventive pattern comprises a non-conductive pattern 22 and a conductive pattern 26. For instance, with the aid of the non-conductive pattern 22, the respective product type and/or the size of the product may be shown.

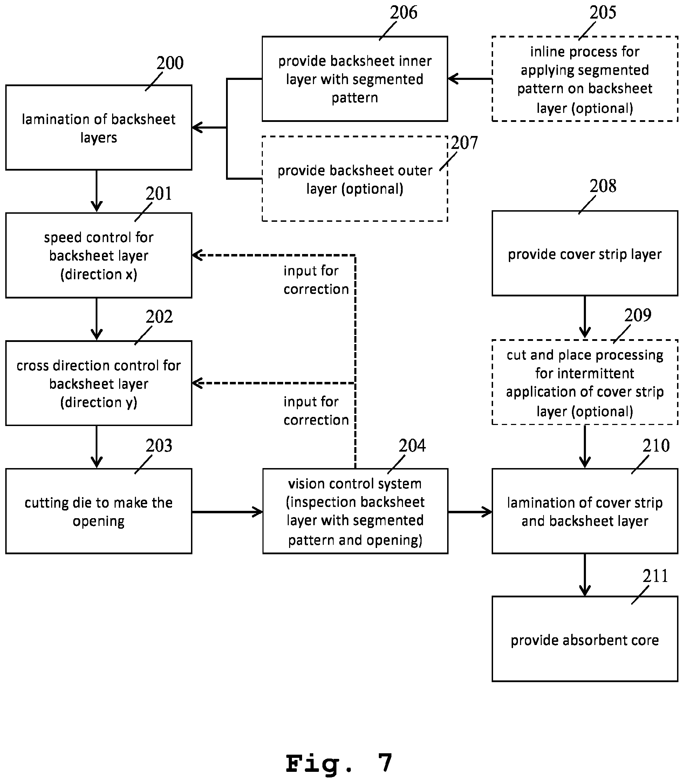

Finally, Fig. 7 presents a flow chart of an exemplary embodiment of the inventive manufacturing method in greater detail.

As it can be seen from Fig. 7, the method comprises the step of a lamination of backsheet layers 200.

Before said lamination step 200, a backsheet inner layer with the inventive pattern, exemplarily a segmented pattern, is provided according to step 206. In this context, it is noted that there may exist an optional inline process for applying said pattern or segmented pattern, respectively, on the backsheet layer or the backsheet inner layer, respectively, in accordance with the optional step 205.

Further optionally, before the lamination step 200, a backsheet outer layer may be provided according to step 207.

After said lamination step 200, the speed for the backsheet layer is controlled according to step 201 especially with the aid of the inventive first offset value (x). Furthermore, in accordance with step 202, the cross direction for the backsheet is controlled especially with the aid of the inventive second offset value (y).

Moreover, in accordance with step 203, a cutting die is exemplarily used to make or cut the opening. It is further noted that according to step 204, a vision control system may be used for an inspection of the backsheet layer with the inventive pattern, exemplarily the segmented pattern, and the inventive opening.

Said vision control system may especially provide the input for correction, exemplarily the first offset value x, for the above-mentioned step 201 and/or the input for correction, exemplarily the second offset value y, for the abov-mentioned step 202.

Furthermore, according to step 208, a cover strip layer is provided. With respect to said cover strip layer, a cut and place processing may optionally be performed for an intermittent application of the cover strip layer in accordance with the optional step 209.

Moreover, before an absorbent core is provided according to step 211, a lamination of the cover strip layer and the backsheet layer is performed in accordance with step 210.

While various embodiments of the present invention have been described above, it should be understood that they have been presented by way of example only, and not limitation. Numerous changes to the disclosed embodiments can be made in accordance with the disclosure herein without departing from the scope of the invention. Thus, the breadth and scope of the present invention should not be limited by any of the above described embodiments. Rather, the scope of the invention should be defined in accordance with the following claims.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A manufacturing method for manufacturing an absorbent article (17, 18) comprising a backsheet layer (11) on the garment facing side of said absorbent article (17, 18) and a pocket being accessible from the garment facing side of the absorbent article (17, 18) through an opening of the backsheet layer (11), the manufacturing method comprising the steps of:
providing a backsheet layer (11), a cover strip layer (12), and an absorbent core layer (13),
cutting an opening into the backsheet layer (11), and
placing the cover strip layer (12) between the backsheet layer (11) and the absorbent core layer (13), in such a manner that the opening of the backsheet layer (11) is at least partly covered by the cover strip layer (12), wherein the cutting is speed-synchronized with respect to the provision of the backsheet layer (11) in such a manner that the opening is placed at the same position (21) for every absorbent article (17, 18), or
wherein in the case that the absorbent article (17, 18) further comprises a pattern on the body facing side of the backsheet layer (11), the backsheet layer (11) is speed-controlled with respect to the positioning of the pattern in such a manner that the opening is placed at the same position (21) relatively to the pattern for every absorbent article (17, 18).

2. The manufacturing method according to claim 1,
wherein the pattern is arranged in such manner onto the backsheet layer (11) that the same portion of the pattern is positioned in the same way for every absorbent article (17, 18), and/or
wherein the pattern comprises a conductive pattern especially being applied onto the backsheet layer (11) by means of printing and/or spraying and/or laminating conductive material onto the backsheet layer (11) and/or a non-conductive pattern especially being applied onto the backsheet layer (11) by means of printing and/or embossing.

3. The manufacturing method according to claim 1 or 2,
wherein the pattern comprises an ink, preferably a carbon-based ink and/or a conductive polymer-based ink,
wherein the ink especially comprises at least one of graphene, graphite, nano-carbon-tubes, polyacetylene, polypyrrole, polyaniline and copolymers thereof, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), poly(3,4-ethylenedioxythiophene) polystyrene sulfonate (PEDOT:PSS), or any combination thereof.

4. The manufacturing method according to any of the claims claim 1 to 3,
wherein the pattern is applied onto the backsheet layer (11) prior to converting the backsheet layer (11), or
wherein the pattern is applied within the same manufacturing process onto the backsheet layer (11) with the aid of a pattern application unit, wherein the pattern application unit is especially synchronized at least with the process step of cutting the opening into the backsheet layer (11).

5. The manufacturing method according to any of the claims 1 to 4,
wherein with the aid of a visual control unit, the positioning of the pattern on the backsheet layer (11) relatively to the opening of said backsheet layer (11) is visually controlled to determine a first offset value (x) and/or a second offset value (y) by processing visual inspection data,
wherein the first offset value (x) is especially adapted to quantify the deviation of said positioning in converting direction of the backsheet layer (11), and/or
wherein the second offset value (y) is especially adapted to quantify the deviation of said positioning in the direction orthogonal to the converting direction of the backsheet layer (11), and/or
wherein the visual control unit especially comprises a camera and/or an optical sensor.

6. The manufacturing method according to claim 5,
wherein the first offset value (x) is used to drive the speed-control of the backsheet layer (11).

7. The manufacturing method according to claim 5 or 6, wherein the second offset value (y) is used to move the backsheet layer (11) with the pattern relatively to the opening and relatively to the cover strip layer (12) in a direction perpendicular to the converting direction in such a manner that the second offset value (y) is reduced towards zero, wherein the relative positioning of said backsheet layer (11) is especially driven by means of a web guiding device for the backsheet layer (11) and / or by a web guiding device for the cover strip layer (12) and / or by a device to move a corresponding cutting device for cutting the opening in a direction orthogonal to the converting direction.

8. The manufacturing method according to any of the claims 1 to 7,
wherein the opening of the backsheet layer (11) is cut with the aid of at least one of a rotary knife, a cutting die, a punching tool, a laser cutting tool, a water jet cutting tool, or any combination thereof, and/or
wherein the cover strip layer (12) is placed between the backsheet layer (11) and the absorbent core layer (13) in such a manner that the opening of the backsheet layer (11) is at least partly covered by the cover strip layer (12).

9. The manufacturing method according to any of the claims 1 to 8,
wherein the width of the cover strip layer (12) is less than the width of the backsheet layer (11) and/or the width of the absorbent article (13), and/or
wherein the width of the cover strip layer (12) is wider than the opening of the backsheet layer (11), and/or wherein the cover strip layer (12) comprises or is a liquid barrier.

10. The manufacturing method according to any of the claims 1 to 9,
wherein the cover strip layer (12) is non-intermittently placed, or
wherein the cover strip layer (12) is intermittently placed,
wherein the intermittent placement of the cover strip layer (12) comprises the additional step of cutting the cover strip layer (12) in such a manner that the length of the cover strip layer (12) is less than the length of the backsheet layer (11) and/or the length of the absorbent article (13).

11. The manufacturing method according to any of the claims 1 to 10,
wherein the manufacturing method comprises the step of sealing at least two edges, preferably four edges, of the cover strip layer (12) to the backsheet layer (11) especially in such a manner that the sealing pattern is surrounding the opening to form the pocket between said backsheet layer (11) and said cover strip layer (12).

12. The manufacturing method according to claim 11, wherein the step of sealing comprises an adhesive application and/or a welding, preferably an ultrasonic welding, application.

13. The manufacturing method according to any of the claims 1 to 12,
wherein the backsheet layer (11) comprises at least one outer layer (11a) and at least one inner layer (11b) being closer to the cover strip layer (12) than the at least one outer layer (11a),
wherein at least two, preferably all, layers (11a, 11b) of the backsheet layer (11) are especially laminated to each other, and/or
wherein the at least one outer layer (11a) especially covers at least a part of the surface of the at least one inner layer (11b) and/or especially at least surrounds the opening of the backsheet layer (11).

14. The manufacturing method according to claim 10,
wherein the at least one outer layer (11a) comprises a substrate, preferably a non-woven substrate, and/or
wherein the at least one inner layer (11b) comprises a film, preferably a non-permeable film.

15. An absorbent article comprising a backsheet layer, a cover strip layer, an absorbent core layer, and a pocket being accessible from the non-absorbent side of the absorbent article, the absorbent article being producable by the steps of a method of one of the claims 1 to 14, wherein the method comprises the steps of cutting an opening into the backsheet layer (11), and placing the cover strip layer (12) between backsheet layer (11) and the absorbent core layer (13), in such a manner that the opening of the backsheet layer (11) is at least partly covered by the cover strip layer (12), wherein the width of the cover strip layer (12) is less than the width of the backsheet layer (11) and/or the width of the absorbent article (13), and/or
wherein the width of the cover strip layer (12) is wider than the opening of the backsheet layer (11),
and
wherein the cover strip layer (12) comprises or is a liquid barrier.

## Patentansprüche

1. Herstellungsverfahren zum Herstellen eines saugfähigen Artikels (17, 18), der eine Rückseitenschicht (11) auf der der Kleidung zugewandten Seite des saugfähigen Artikels (17, 18) und eine Tasche umfasst, die von der der Kleidung zugewandten Seite des saugfähigen Artikels (17, 18) durch eine Öffnung der Rückseitenschicht (11) zugänglich ist, wobei das Herstellungsverfahren die Schritte umfasst:
Bereitstellen einer Rückseitenschicht (11), einer Abdeckstreifenschicht (12) und einer saugfähigen Kernschicht (13),
Schneiden einer Öffnung in die Unterschicht (11), und
Platzieren der Abdeckstreifenschicht (12) zwischen der Rückseitenschicht (11) und der absorbierenden Kernschicht (13), derart, dass die Öffnung der Rückseitenschicht (11) zumindest teilweise durch die Abdeckstreifenschicht (12) abgedeckt ist, wobei das Schneiden geschwindigkeitssynchronisiert mit dem Bereitstellen der Rückseitenschicht (11) derart erfolgt, dass die Öffnung bei jedem absorbierenden Artikel (17, 18) an der gleichen Stelle (21) platziert wird, oder
wobei in dem Fall, dass der saugfähige Artikel (17, 18) ferner ein Muster auf der dem Körper zugewandten Seite der Rückseitenschicht (11) umfasst, die Rückseitenschicht (11) hinsichtlich der Positionierung des Musters geschwindigkeitsgesteuert ist, und zwar in der Weise, dass die Öffnung bei jedem saugfähigen Artikel (17, 18) an der gleichen Position (21) relativ zum Muster platziert ist.

2. Herstellungsverfahren nach Anspruch 1,
wobei das Muster so auf der Rückseitenschicht (11) angeordnet ist, dass der gleiche Teil des Musters bei jedem absorbierenden Artikel (17, 18) in der gleichen Weise positioniert ist, und/oder
wobei das Muster ein leitfähiges Muster umfasst, das insbesondere durch Drucken und/oder Sprühen und/oder Laminieren von leitfähigem Material auf die Rückseitenschicht (11) aufgebracht wird, und/oder ein nicht leitfähiges Muster, das insbesondere durch Drucken und/oder Prägen auf die Rückseitenschicht (11) aufgebracht wird.

3. Herstellungsverfahren nach Anspruch 1 oder 2,
wobei das Muster eine Tinte umfasst, vorzugsweise eine kohlenstoffbasierte Tinte und/oder eine leitfähige polymerbasierte Tinte,
wobei die Tinte insbesondere mindestens eines von Graphen, Graphit, Nano-Kohlenstoffröhren, Polyacetylen, Polypyrrol, Polyanilin und Copolymere davon, Polypyrrole (PPY), Polyaniline (PANI), Polythiophene (PT), Polypphenylensulfid (PPS), Polypphenylen (PPP), Polyacetylene (PAC), Polypphenylenvinylen (PPV), Poly(3,4-ethylendioxythiophen (PEDOT), Poly(3,4-ethylendioxythiophen)-Polystyrolsulfonat (PEDOT:PSS) oder eine beliebige Kombination davon umfasst.

4. Herstellungsverfahren nach einem der Ansprüche 1 bis 3,
wobei das Muster vor dem Umwandeln der Rückseitenschicht (11) auf die Rückseitenschicht (11) aufgebracht wird oder
wobei das Muster innerhalb desselben Herstellungsprozesses mit Hilfe einer Musteraufbringeinheit auf die Rückseitenschicht (11) aufgebracht wird, wobei die Musteraufbringeinheit insbesondere zumindest mit dem Prozessschritt des Schneidens der Öffnung in die Rückseitenschicht (11) synchronisiert ist.

5. Herstellungsverfahren nach einem der Ansprüche 1 bis 4,
wobei mit Hilfe einer visuellen Kontrolleinheit die Positionierung des Musters auf der Rückseitenschicht (11) relativ zur Öffnung der Rückseitenschicht (11) visuell kontrolliert wird, um durch Verarbeitung visueller Inspektionsdaten einen ersten Versatzwert (x) und/oder einen zweiten Versatzwert (y) zu bestimmen,
wobei der erste Versatzwert (x) insbesondere dazu geeignet ist, die Abweichung der Positionierung in Verarbeitungsrichtung der Rückseitenschicht (11) zu quantifizieren, und/oder
wobei der zweite Versatzwert (y) insbesondere dazu geeignet ist, die Abweichung der Positionierung in der Richtung orthogonal zur Umwandlungsrichtung der Rückseitenschicht (11) zu quantifizieren, und/oder
wobei die visuelle Kontrolleinheit insbesondere eine Kamera und/oder einen optischen Sensor umfasst.

6. Herstellungsverfahren nach Anspruch 5,
wobei der erste Offsetwert (x) zum Ansteuern der Geschwindigkeitsregelung der Rückseitenschicht (11) verwendet wird.

7. Herstellungsverfahren nach Anspruch 5 oder 6,
wobei der zweite Versatzwert (y) verwendet wird, um die Rückseitenschicht (11) mit dem Muster relativ zu der Öffnung und relativ zu der Abdeckstreifenschicht (12) in einer Richtung senkrecht zur Verarbeitungsrichtung derart zu bewegen, dass der zweite Versatzwert (y) gegen Null reduziert wird, wobei die relative Positionierung der Rückseitenschicht (11) insbesondere mittels einer Bahnführungsvorrichtung für die Rückseitenschicht (11) und/oder durch eine Bahnführungsvorrichtung für die Abdeckstreifenschicht (12) und/oder durch eine Vorrichtung zum Bewegen einer entsprechenden Schneidvorrichtung zum Schneiden der Öffnung in einer Richtung orthogonal zur Verarbeitungsrichtung angetrieben wird.

8. Herstellungsverfahren nach einem der Ansprüche 1 bis 7,
wobei die Öffnung der Rückseitenschicht (11) mit Hilfe von mindestens einem Rotationsmesser, einer Schneideform, einem Stanzwerkzeug, einem Laserschneidwerkzeug, einem Wasserstrahlschneidwerkzeug oder einer beliebigen Kombination davon geschnitten wird, und/oder
wobei die Abdeckstreifenschicht (12) zwischen der Rückseitenschicht (11) und der absorbierenden Kernschicht (13) derart angeordnet ist, dass die Öffnung der Rückseitenschicht (11) zumindest teilweise durch die Abdeckstreifenschicht (12) abgedeckt ist.

9. Herstellungsverfahren nach einem der Ansprüche 1 bis 8,
wobei die Breite der Abdeckstreifenschicht (12) geringer ist als die Breite der Rückseitenschicht (11) und/oder die Breite des absorbierenden Artikels (13), und/oder
wobei die Breite der Abdeckstreifenschicht (12) größer ist als die Öffnung der Rückseitenschicht (11), und/oder
wobei die Abdeckstreifenschicht (12) eine Flüssigkeitsbarriere umfasst oder ist.

10. Herstellungsverfahren nach einem der Ansprüche 1 bis 9,
wobei die Abdeckstreifenschicht (12) nicht unterbrochen angeordnet ist oder
wobei die Abdeckstreifenschicht (12) intermittierend platziert wird, wobei das intermittierende Platzieren der Abdeckstreifenschicht (12) den zusätzlichen Schritt des Schneidens der Abdeckstreifenschicht (12) in einer solchen Weise umfasst, dass die Länge der Abdeckstreifenschicht (12) kürzer ist als die Länge der Rückseitenschicht (11) und/oder die Länge des absorbierenden Artikels (13).

11. Herstellungsverfahren nach einem der Ansprüche 1 bis 10,
wobei das Herstellungsverfahren den Schritt des Versiegelns von mindestens zwei Kanten, vorzugsweise vier Kanten, der Abdeckstreifenschicht (12) mit der Rückseitenschicht (11) umfasst, insbesondere in der Weise, dass das Versiegelungsmuster die Öffnung umgibt, um die Tasche zwischen der Rückseitenschicht (11) und der Abdeckstreifenschicht (12) zu bilden.

12. Herstellungsverfahren nach Anspruch 11,
wobei der Schritt des Versiegelns einen Klebstoffauftrag und/oder einen Schweißauftrag, vorzugsweise einen Ultraschallschweißauftrag, umfasst.

13. Herstellungsverfahren nach einem der Ansprüche 1 bis 12,
wobei die Rückseitenschicht (11) mindestens eine Außenschicht (11a) und mindestens eine Innenschicht (11b) umfasst, die näher an der Abdeckstreifenschicht (12) liegt als die mindestens eine Außenschicht (11a),
wobei mindestens zwei, vorzugsweise alle Lagen (11a, 11b) der Rückseitenschicht (11) miteinander insbesondere laminiert sind, und/oder
wobei die mindestens eine Außenschicht (11a) insbesondere zumindest einen Teil der Oberfläche der mindestens einen Innenschicht (11b) bedeckt und/oder insbesondere zumindest die Öffnung der Rückseitenschicht (11) umgibt.

14. Herstellungsverfahren nach Anspruch 10,
wobei die mindestens eine Außenschicht (11a) ein Substrat, vorzugsweise ein Vliessubstrat, umfasst, und/oder
wobei die mindestens eine Innenschicht (11b) eine Folie, vorzugsweise eine undurchlässige Folie, umfasst.

15. Saugfähiger Artikel, umfassend eine Rückseitenschicht, eine Deckstreifenschicht, eine saugfähige Kernschicht und eine Tasche, die von der nicht saugfähigen Seite des saugfähigen Artikels aus zugänglich ist, wobei der saugfähige Artikel durch die Schritte eines Verfahrens nach einem der Ansprüche 1 bis 14 herstellbar ist, wobei das Verfahren die Schritte des Schneidens einer Öffnung in die Rückseitenschicht (11) und des Platzierens der Deckstreifenschicht (12) zwischen der Rückseitenschicht (11) und der saugfähigen Kernschicht (13) umfasst, und zwar in einer solchen Weise, dass die Öffnung der Rückseitenschicht (11) zumindest teilweise durch die Deckstreifenschicht (12) bedeckt ist, wobei die Breite der Deckstreifenschicht (12) geringer ist als die Breite der Rückseitenschicht (11) und/oder die Breite des saugfähigen Artikels (13), und/oder
wobei die Breite der Abdeckstreifenschicht (12) größer ist als die Öffnung der Rückseitenschicht (11),
und
wobei die Abdeckstreifenschicht (12) eine Flüssigkeitsbarriere umfasst oder ist.

## Revendications

1. Procédé de fabrication permettant de fabriquer un article absorbant (17, 18) comprenant une couche de feuille de fond (11) sur le côté dudit article absorbant (17, 18) tourné vers le vêtement et une poche qui est accessible depuis le côté de l'article absorbant (17, 18) tourné vers le vêtement à travers une ouverture de la couche de feuille de fond (11), le procédé de fabrication comprenant les étapes consistant à :
fournir une couche de feuille de fond (11), une couche de bande de recouvrement (12), et une couche centrale absorbante (13),
découper une ouverture dans la couche de feuille de fond (11), et
placer la couche de bande de recouvrement (12) entre la couche de feuille de fond (11) et la couche centrale absorbante (13), d'une manière telle que l'ouverture de la couche de feuille de fond (11) est au moins partiellement recouverte par la couche de bande de recouvrement (12), dans lequel la découpe est synchronisée en vitesse par rapport à la fourniture de la couche de feuille de fond (11) d'une manière telle que l'ouverture est placée à la même position (21) pour chaque article absorbant (17, 18), ou
dans lequel, dans le cas où l'article absorbant (17, 18) comprend en outre un motif sur le côté de la couche de feuille de fond (11) tourné vers le corps, la couche de feuille de fond (11) est régulée en vitesse par rapport au positionnement du motif d'une manière telle que l'ouverture est placée à la même position (21) par rapport au motif pour chaque article absorbant (17, 18).

2. Procédé de fabrication selon la revendication 1,
dans lequel le motif est disposé d'une manière telle sur la couche de feuille de fond (11) que la même partie du motif est positionnée de la même manière pour chaque article absorbant (17, 18), et/ou
dans lequel le motif comprend un motif conducteur appliqué en particulier sur la couche de feuille de fond (11) au moyen d'une impression et/ou d'une pulvérisation et/ou d'un laminage d'un matériau conducteur sur la couche de feuille de fond (11) et/ou un motif non conducteur appliqué en particulier sur la couche de feuille de fond (11) au moyen d'une impression et/ou d'un gaufrage.

3. Procédé de fabrication selon la revendication 1 ou 2,
dans lequel le motif comprend une encre, de préférence une encre à base de carbone et/ou une encre à base de polymère conducteur,
dans lequel l'encre comprend en particulier au moins l'un parmi graphène, graphite, nanotubes de carbone, polyacétylène, polypyrrole, polyaniline et copolymères de ceux-ci, poly(pyrrole)s (PPY), polyanilines (PANI), poly(thiophène)s (PT), poly(sulfure de p-phénylène) (PPS), poly(p-phénylène) (PPP), poly(acétylène)s (PAC), poly(p-phénylène vinylène) (PPV), poly(3,4-éthylènedioxythiophène) (PEDOT), poly(3,4-éthylènedioxythiophène) polystyrène sulfonate (PEDOT :PSS), ou toute combinaison de ceux-ci.

4. Procédé de fabrication selon l'une quelconque des revendications 1 à 3,
dans lequel le motif est appliqué sur la couche de feuille de fond (11) avant de convertir la couche de feuille de fond (11), ou
dans lequel le motif est appliqué au cours du même processus de fabrication sur la couche de feuille de fond (11) à l'aide d'une unité d'application de motif, dans lequel l'unité d'application de motif est en particulier synchronisée au moins avec l'étape de processus consistant à découper l'ouverture dans la couche de feuille de fond (11).

5. Procédé de fabrication selon l'une quelconque des revendications 1 à 4,
dans lequel, à l'aide d'une unité de vérification visuelle, le positionnement du motif sur la couche de feuille de fond (11) par rapport à l'ouverture de ladite couche de feuille de fond (11) est vérifié visuellement pour déterminer une première valeur de décalage (x) et/ou une seconde valeur de décalage (y) en traitant des données d'inspection visuelle,
dans lequel la première valeur de décalage (x) est en particulier adaptée pour quantifier l'écart dudit positionnement dans la direction de conversion de la couche de feuille de fond (11), et/ou
dans lequel la seconde valeur de décalage (y) est en particulier adaptée pour quantifier l'écart dudit positionnement dans la direction orthogonale à la direction de conversion de la couche de feuille de fond (11), et/ou
dans lequel l'unité de vérification visuelle comprend en particulier une caméra et/ou un capteur optique.

6. Procédé de fabrication selon la revendication 5,
dans lequel la première valeur de décalage (x) est utilisée pour entraîner la régulation en vitesse de la couche de feuille de fond (11).

7. Procédé de fabrication selon la revendication 5 ou 6,
dans lequel la seconde valeur de décalage (y) est utilisée pour déplacer la couche de feuille de fond (11) avec le motif par rapport à l'ouverture et par rapport à la couche de bande de recouvrement (12) dans une direction perpendiculaire à la direction de conversion d'une manière telle que la seconde valeur de décalage (y) est réduite vers zéro, dans lequel le positionnement relatif de ladite couche de feuille de fond (11) est en particulier entraîné au moyen d'un dispositif de guidage de bande pour la couche de feuille de fond (11) et/ou d'un dispositif de guidage de bande pour la couche de bande de recouvrement (12) et/ou d'un dispositif pour déplacer un dispositif de découpe correspondant permettant de découper l'ouverture dans une direction orthogonale à la direction de conversion.

8. Procédé de fabrication selon l'une quelconque des revendications 1 à 7,
dans lequel l'ouverture de la couche de feuille de fond (11) est découpée à l'aide d'au moins l'un parmi un couteau rotatif, une matrice de découpe, un outil de poinçonnage, un outil de découpe au laser, un outil de découpe au jet d'eau, ou toute combinaison de ceux-ci, et/ou
dans lequel la couche de bande de recouvrement (12) est placée entre la couche de feuille de fond (11) et la couche centrale absorbante (13) d'une manière telle que l'ouverture de la couche de feuille de fond (11) est au moins partiellement recouverte par la couche de bande de recouvrement (12).

9. Procédé de fabrication selon l'une quelconque des revendications 1 à 8,
dans lequel la largeur de la couche de bande de recouvrement (12) est inférieure à la largeur de la couche de feuille de fond (11) et/ou à la largeur de l'article absorbant (13), et/ou
dans lequel la largeur de la couche de bande de recouvrement (12) est supérieure à l'ouverture de la couche de feuille de fond (11), et/ou
dans lequel la couche de bande de recouvrement (12) comprend ou est une barrière contre les liquides.

10. Procédé de fabrication selon l'une quelconque des revendications 1 à 9,
dans lequel la couche de bande de recouvrement (12) est placée de manière non intermittente, ou
dans lequel la couche de bande de recouvrement (12) est placée de manière intermittente, dans lequel la mise en place intermittente de la couche de bande de recouvrement (12) comprend l'étape supplémentaire consistant à découper la couche de bande de recouvrement (12) d'une manière telle que la longueur de la couche de bande de recouvrement (12) est inférieure à la longueur de la couche de feuille de fond (11) et/ou à la longueur de l'article absorbant (13).

11. Procédé de fabrication selon l'une quelconque des revendications 1 à 10,
dans lequel le procédé de fabrication comprend l'étape consistant à sceller au moins deux bords, de préférence quatre bords, de la couche de bande de recouvrement (12) à la couche de feuille de fond (11), en particulier d'une manière telle que le motif de scellement entoure l'ouverture pour former la poche entre ladite couche de feuille de fond (11) et ladite couche de bande de recouvrement (12).

12. Procédé de fabrication selon la revendication 11,
dans lequel l'étape de scellement comprend une application d'adhésif et/ou une application de soudure, de préférence une soudure par ultrasons.

13. Procédé de fabrication selon l'une quelconque des revendications 1 à 12,
dans lequel la couche de feuille de fond (11) comprend au moins une couche externe (11a) et au moins une couche interne (11b) qui est plus proche de la couche de bande de recouvrement (12) que l'au moins une couche externe (11a),
dans lequel au moins deux, de préférence toutes les couches (11a, 11b) de la couche de feuille de fond (11) sont en particulier laminées les unes sur les autres, et/ou
dans lequel l'au moins une couche externe (11a) recouvre en particulier au moins une partie de la surface de l'au moins une couche interne (11b) et/ou en particulier entoure au moins l'ouverture de la couche de la couche de feuille de fond (11).

14. Procédé de fabrication selon la revendication 10,
dans lequel l'au moins une couche externe (11a) comprend un substrat, de préférence un substrat non tissé, et/ou
dans lequel l'au moins une couche interne (11b) comprend un film, de préférence un film non perméable.

15. Article absorbant comprenant une couche de feuille de fond, une couche de bande de recouvrement, une couche centrale absorbante et une poche qui est accessible depuis le côté non absorbant de l'article absorbant, l'article absorbant pouvant être produit par les étapes d'un procédé selon l'une quelconque des revendications 1 à 14, dans lequel le procédé comprend les étapes consistant à découper une ouverture dans la couche de feuille de fond (11), et à placer la couche de bande de recouvrement (12) entre la couche de feuille de fond (11) et la couche centrale absorbante (13), d'une manière telle que l'ouverture de la couche feuille de fond (11) est au moins partiellement recouverte par la couche de bande de recouvrement (12), dans lequel la largeur de la couche de bande de recouvrement (12) est inférieure à la largeur de la couche de feuille de fond (11) et/ou à la largeur de l'article absorbant (13), et/ou
dans lequel la largeur de la couche de bande de recouvrement (12) est supérieure à l'ouverture de la couche de feuille de fond (11),
et
dans lequel la couche de bande de recouvrement (12) comprend ou est une barrière contre les liquides.
